(19) Europäisches Patentamt
European Patent Office
Office européen des brevets

(11) Publication number: 0 163 458
A1

(12) # EUROPEAN PATENT APPLICATION

(21) Application number: 85303472.6

(22) Date of filing: 17.05.85

(51) Int. Cl.[4]: C 07 C 93/14
C 07 C 91/23, C 07 D 209/16
C 07 D 277/64, C 07 D 235/14
C 07 D 263/56
//A61K31/135

(30) Priority: 17.05.84 US 611258

(43) Date of publication of application:
04.12.85 Bulletin 85/49

(84) Designated Contracting States:
AT BE CH DE FR GB IT LI LU NL SE

(71) Applicant: E.R. Squibb & Sons, Inc.
Lawrenceville-Princeton Road
Princeton, N.J. 08540(US)

(72) Inventor: Atwal, Karnail S.
2634 Old Stone Mill Drive
Cranbury New Jersey(US)

(74) Representative: Thomas, Roger Tamlyn et al,
D. Young & Co. 10 Staple Inn
London WC1V 7RD(GB)

(54) **Tetrahydronaphthalenols for the treatment of hypertension.**

(57) New compounds, useful for the treatment of hypertension, have the formula

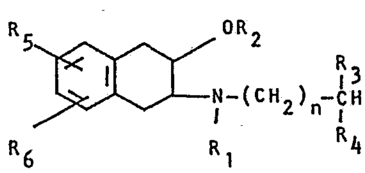

and pharmaceutically acceptable salts thereof, wherein

$R_1$ is hydrogen or methyl;

$R_2$ is hydrogen, alkyl, alkanoyl, or arylcarbonyl;

$R_3$ and $R_4$ are each aryl; $R_3$ and $R_4$ are each cycloalkyl; $R_3$ is hydrogen or alkyl and $R_4$ is cycloalkyl or 9H-fluoren-9-yl; or $R_3$ is hydrogen or alkyl and $R_4$ is heteroaryl;

$R_5$ and $R_6$ are the same or different and each is hydrogen, hydroxy, alkoxy or alkanoyl; and

n is 1, 2, 3 and 4.

EP 0 163 458 A1

# TETRAHYDRONAPHTHALENOLS FOR THE TREATMENT OF HYPERTENSION

This invention relates to new compounds having the formula

I 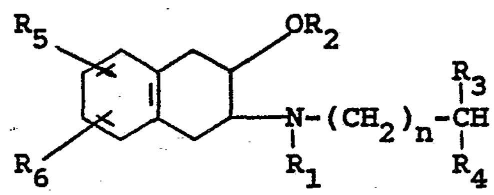 ,

and pharmaceutically acceptable salts thereof, which are useful for the treatment of hypertension. In formula I, and throughout the specification, the symbols are as defined below.

$R_1$ is hydrogen or methyl;

$R_2$ is hydrogen, alkyl, alkanoyl, or arylcarbonyl;

$R_3$ and $R_4$ are each aryl; $R_3$ and $R_4$ are each cycloalkyl; $R_3$ is hydrogen or alkyl and $R_4$ is cycloalkyl or 9H-fluoren-9-yl; or $R_3$ is hydrogen or alkyl and $R_4$ is heteroaryl;

$R_5$ and $R_6$ are the same or different and each is hydrogen, hydroxy, alkoxy or alkanoyl; and

n is 1, 2, 3 or 4.

The terms "alkyl" and "alkoxy", as used throughout the specification, refer to groups having 1 to 7 carbon atoms.

The term "cycloalkyl", as used throughout the specification, refers to cycloalkyl groups having 3, 4, 5, 6 or 7 carbon atoms.

The term "alkanoyl", as used throughout the specification, refers to alkanoyl groups having 2 to 7 carbon atoms.

The term "aryl", as used throughout the specification, refers to phenyl and phenyl substituted with 1, 2, or 3 alkyl, alkoxy, halogen, alkylamino, dialkylamino, alkanoylamino or arylcarbonylamino groups.

The term "heteroaryl", as used throughout the specification, refers to 2-, 3- or 4-pyridinyl, 2- or 3-furanyl, 2- or 3-thienyl, 1-, 2- or 3-indolyl, 2-benzoxazolyl, 2-benzothiazolyl, 1- or 2-benzimidazolyl, 2- or 3-benzothienyl and 2- or 3-benzofuranyl, and to any of the above groups substituted with 1, 2 or 3 alkyl, alkoxy, halogen, alkylamino, dialkylamino, alkanoylamino, or arylcarbonylamino groups.

United States Patent 3,930,022, issued December 30, 1975, discloses as beta-adrenergic receptor blocking agents 5,6,7,8-tetrahydronaphthalenes having the formula

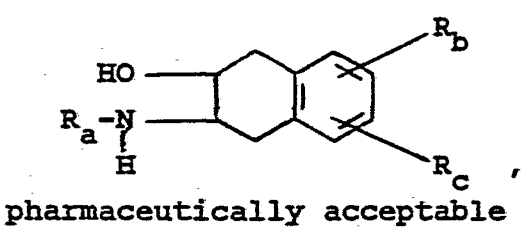

and pharmaceutically acceptable acid-addition salts thereof, wherein $R_a$ is alkyl or arylalkyl, and $R_b$ and $R_c$ can each be hydrogen, hydroxyl, alkoxy or arylalkoxy.

United States Patents 4,076,843, issued February 8, 1978, and 4,267,373, issued May 12, 1981, disclose as hypotensive agents 5,6,7,8-tetrahydronaphthalenes having the formula

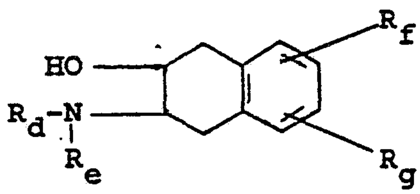,

and pharmaceutically acceptable salts thereof, wherein $R_d$ is hydrogen or alkyl, $R_e$ is alkyl, and $R_f$ and $R_g$ can each be hydrogen, hydroxyl, alkoxy, or arylalkoxy, but at least one of $R_f$ and $R_g$ must be other than hydroxyl.

United States Patent 4,081,444, issued March 28, 1978, discloses as hypotensive agents 5,6,7,8-tetrahydronaphthalenes having the formula

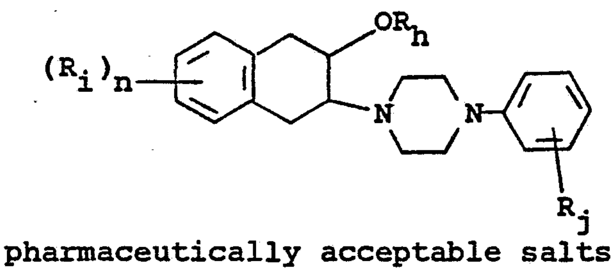,

and pharmaceutically acceptable salts thereof, wherein $R_h$ is hydrogen, $X-\overset{O}{\overset{\|}{C}}-$ (wherein X is alkyl of 1 to 12 carbon atoms or aryl) or $Y-NH-\overset{O}{\overset{\|}{C}}-$ (wherein Y is lower alkyl or aryl), $R_i$ is hydrogen, halogen, lower alkyl, alkoxy, or $X-\overset{O}{\overset{\|}{C}}-$, $R_j$ is hydrogen, halogen, lower alkyl, alkoxy, alkylthio, trifluoromethyl, or nitro, and n is 1, 2 or 3.

The compounds of this invention wherein $R_2$ is hydrogen can be prepared by reacting a

1,2,3,4-tetrahydro-6,7-epoxynaphthalene having the formula

II

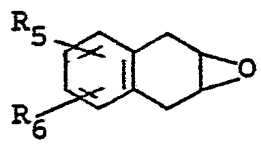

with an amine having the formula

III

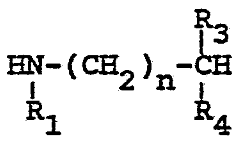

The reaction proceeds most readily in a high boiling alcoholic solvent (preferably at 150-160°C) or in methylene chloride with trialkyl aluminum as a catalyst (preferably at room temperature), and yields a compound having the formula

IV

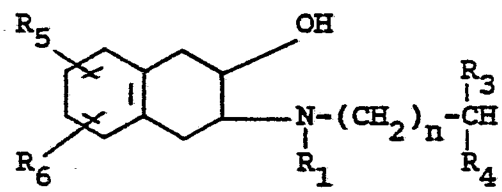

The compounds of formula I wherein $R_2$ is alkanoyl or arylcarbonyl can be obtained by acylating a strong acid salt (e.g., the hydrochloride, hydrobromide or hydrogen sulfate salt) of the corresponding compound of formula IV. Standard acylation techniques can be used, but preferably an acyl halide will be used as the acylating agent.

Both the cis and the trans isomers of the compounds of formula I are specifically contemplated for use in this invention. The above-described methodology yields the trans compounds. The desired cis products can be prepared from the

corresponding cis isomer of a compound having the formula

V

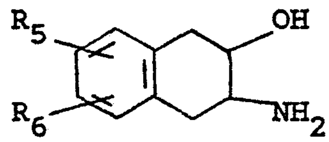

.

Methodology for preparing cis compounds of formula V is known; see, for example, United States patent 4,081,444, issued March 28, 1978. As described therein, the trans isomer of a compound of formula V is reacted with an acyl chloride (e.g., benzoyl chloride) in aqueous base to give the corresponding acylamido alcohol. The acylamido alcohol is converted to the cis isomer of a compound of formula V by sequential reaction with thionyl chloride and a mineral acid.

The cis isomers of the products of formula I can be prepared by reductively alkylating a cis isomer of a compound of formula V with the appropriate aldehyde or ketone having the formula

VI

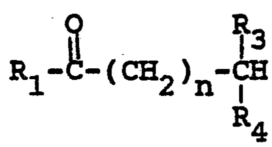

,

using a reducing agent such as sodium cyanoborohydride or using catalytic hydrogenation.

The compounds of formula I form acid addition salts with inorganic and organic acids. These acid addition salts frequently provide useful means for isolating the products from reaction mixtures by forming the salt in a medium in which it is insoluble. The free base may then be obtained by neutralization, e.g., with a base such as sodium hydroxide. Then any other salt may again be formed from the free base and the appropriate inorganic or organic acid. Illustrative are the hydrohalides, especially the

hydrochloride and hydrobromide which are preferred, sulfate, nitrate, phosphate, borate, acetate, tartrate, maleate, citrate, succinate, benzoate, ascorbate, salicylate, methanesulfonate, benzenesulfonate, toluenesulfonate and the like.

The compounds of formula I, and the pharmaceutically acceptable salts thereof, are used for lowering the blood pressure of a hypertensive host. Daily doses of from about 10 milligrams to about 1.0 gram can be administered in single or divided doses. The particular daily dosage used will very with the potency of the particular compound used and with the severity of a particular patient's hypertensive condition.

The active compounds of the present invention can be administered orally, for example, with an inert diluent or with an assimilable edible carrier, or they can be enclosed in hard or soft gelatin capsules, or they can be compressed into tablets, or they can be incorporated directly with the food of the diet. For oral therapeutic administration, the active compounds of this invention can be incorporated with excipients and used in the form of tablets, troches, capsules, elixirs, suspensions, syrups, wafers, chewing gum, and the like. Such compositions and preparations should contain at least 0.1% of active compound. The percentage in the compositions and preparations can, of course, be varied and can conveniently be between about 5% to about 75% or more of the weight of the unit. The amount of active compound in such therapeutically useful compositions or preparations is such that a suitable dosage will be obtained. Preferred compositions or preparations according to

the present invention are prepared so that an oral dosage unit form contains between about 5 and 250 milligrams of active compound.

Without limiting the scope of this invention to any particular mechanism of action, it is believed that the compounds of formula I, and the pharmaceutically acceptable salts thereof, act as vasodilators. In addition to their utility as hypotensive agents, they can also be used to treat migraine headaches, angina, and glaucoma.

Methodology for preparing the 1,2,3,4-tetrahydro-6,7-epoxynaphthalene starting compounds of formula II is described in the literature; see, for example, United States Patents 3,930,022, 4,076,843, 4,081,444, and 4,267,373.

Preferred compounds of this invention are those compounds (and salts thereof) having the formulas

VII

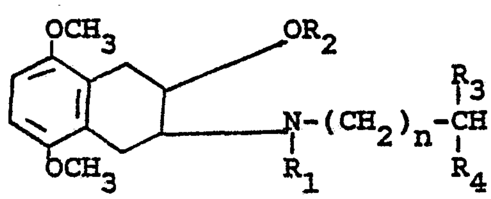

and

VIII

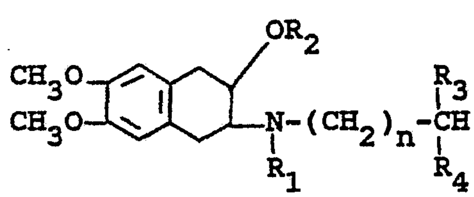

The following examples are specific embodiments of this invention.

## Example 1

trans-1,2,3,4-Tetrahydro-3-[[2-(1H-indol-3-yl)-ethyl]amino]-5,8-dimethoxy-2-naphthalenol

A dark brown solution of 14.4 g (70.0 mmol) of 6,7-epoxy-5,6,7,8-tetrahydro-1,4-dimethoxynaphthalene, 11.6 g (72.3 mmol) of tryptamine, 5.4 ml (~1 eq.) of isopropanol, and 105 ml of xylene was refluxed overnight under argon. The solvent was removed in vacuo and the foamy brown residue was triturated with ether to give ~20 g of an off-white solid. Recrystallization from chloroform gave ~12 g of white crystals. Recrystallization of this material from ethyl acetate failed to give a correct elemental analysis of crystalline needles. A second recrystallization from ethyl acetate gave 918 g of white crystals which did not analyze properly after heating overnight in a vacuum oven at 60°C, 1.5 mm of Hg. 100MHz NMR (deuterated methanol) showed ethyl acetate present. Recrystallization from methanol gave the analytical sample (9.1 g) as granular white crystals, melting point 157-160°C.

Analysis Calc'd. for $C_{22}H_{26}N_2O_3$: C, 72.11; H, 7.15; N, 7.64

Found: C, 72.14; H, 7.17; N, 7.69

## Example 2

trans-1,2,3,4-Tetrahydro-3-[(3,3-diphenylpropyl)-amino]-2-naphthalenol, hydrochloride salt (1:1)

To a brown solution of 3,3-diphenylpropylamine (2.20 g, 10 mmol) in dry dichloromethane (15 ml) under argon was added dropwise triethyl-aluminum, 25.3% solution in benzene (1.90 M, 5.3 ml, 10 mmol); effervescence was observed. The solution was allowed to stir for one hour. 1,2-Epoxy-1,2,3,4-tetrahydronaphthalene (1.46 g; 10 mmol) was then added and the resulting solution was allowed to stir overnight. The reaction mixture was diluted with ~25 ml chloroform and 6 N sodium hydroxide (10 ml) was added dropwise with vigorous stirring. Effervescence was observed and the solvent refluxed briefly. The resulting two phase solution was stirred for one hour. The layers were separated and the aqueous phase was extracted again with chloroform. The combined organic layers were washed with brine, dried over anhydrous potassium carbonate, and rotary evaporated to an off-white solid. Flash chromatography (3% methanol in dichloromethane) followed by treatment with excess ethanolic hydrogen chloride gave white crystals, 2.0 g. This material was combined with 1.5 g from another run and recrystallized from isopropanol containing a trace of methanol. An NMR of the resulting white crystals (2.3 g, melting point 239-241°C showed a doublet at 1.16 ppm corresponding to ~0.1 mole of isopropanol. Heating at 80-90°C under vacuum for 4 hours did not remove the isopropanol, so this 2.3 g was combined with 0.75 g of other material (obtained by recrystallizing the concentrated mother liquors from acetonitrile and recrystallized from acetonitrile) containing a trace of methanol. Analytically pure white crystals (2.7 g, melting point 239-241°C) were obtained.

Analysis Calc'd. for $C_{25}H_{27}NO \cdot HCl$: C, 76.22; H, 7.16; N, 3.56; Cl, 9.00

Found: C, 76.19; H, 7.16; N, 3.59; Cl, 8.95

## Example 3

(*trans*)-3-[(3,3-Diphenylpropyl)amino]-1,2,3,4-tetrahydro-5,8-dimethoxy-2-naphthalenol, hydrochloride salt (1:1)

A solution of 6,7-epoxy-5,6,7,8-tetrahydro-1,4-dimethoxynaphthalene (2.06 g; 10 mmol), 3,3-diphenylpropylamine (2.64 g; 12.5 mmol) and isopropanol (2.30 ml; ~30 mmol, added in three portions at 15 hour intervals) in xylene (15 ml) was heated under reflux for 60 hours (TLC still showed the starting material). The reaction was cooled to room temperature and the solvent was evaporated under reduced pressure to give a brown oily residue. This was dissolved in anhydrous ether (40 ml) and treated with ethanolic hydrogen chloride (5 ml of 5.5 N solution). A colorless thick precipitate of the product as well as the starting amine was formed. It was filtered off and washed with ether to give 4.01 g of white solid. This was dissolved in 150 ml of hot absolute ethanol and the resulting solution was allowed to cool gradually. The hydrochloride of the product crystallized out leaving the 3,3-diphenylpropylamine hydrochloride in solution. The colorless crystals were filtered off, washed with absolute ethanol and ether to give the desired product (1.8 g). Another crystallization from hot ethanol (90 ml) gave analytically pure sample, melting point 249-250°C (dec).

Analysis Calc'd. for $C_{27}H_{31}NO_3 \cdot HCl$: C, 71.43; H, 7.10; N, 3.09

Found: C, 71.24; H, 7.33; N, 3.10

Example 4

(trans)-1,2,3,4-Tetrahydro-3-[[2-(1H-indol-3-yl)-ethyl]amino]-2-naphthalenol

A brown solution of 1.75 g (12.0 mmol) of 2,3-epoxy-1,2,3,4-tetrahydronaphthalene, 2.00 g (12.5 mmol) of tryptamine and 0.92 ml (12 mmol) of isopropanol, in 25 ml of xylene was refluxed under argon for 25 hours. The solution was allowed to cool to room temperature. After evaporation *in vacuo* of a small amount (~5 ml) of solvent, crystals formed. Roughly 5 ml more solvent was evaporated, and the crystals were filtered off and washed with ether a few times. Yield: 1.99 g of off-white crystals. TLC, silica gel, Rf major spot = 0.21 in dichloromethane/methanol/acetic acid (18/1/1). A small contaminant, (probably tryptamine) was visible just above the baseline. An attempt to obtain a second crop gave 100 mg of an off-white solid that showed a slightly larger amount of the contaminant by TLC. The two crops were combined and recrystallized from acetonitrile to give 1.53 g of yellowish needles, melting point 146-151°C. A second recrystallization, from isopropyl ether gave 894 mg first crop, 277 mg second crop, both colorless crystals. First crop melting point 145-148°C.

Analysis Calc'd. for $C_{20}H_{22}N_2O$: C, 78.40; H, 7.24; N, 9.14

Found: C, 78.34; H, 7.34; N, 9.07 (first crop)

### Example 5

(trans)-3-[(2,2-Diphenylethyl)amino]-1,2,3,4-tetrahydro-5,8-dimethoxy-2-naphthalenol, hydrochloride salt (1:1)

The reaction mixture containing 6,7-epoxy-5,6,7,8-tetrahydro-1,4-dimethyoxynaphthalene (2.06 g; 10 mmol), 2,2-diphenylethylamine (2.03 g; 10 mmol) 3-pentanol (1.76 g; 20 mmol) and xylene (10 ml) was heated under reflux (oil bath 145-150°C) for 40 hours. It was cooled to room temperature, and the solvent was evaporated *in vacuo* to give a brown oily residue. This was taken up in ether-methanol (30 ml, 4:1) and treated with ethanolic hydrogen chloride (5 ml of 5.5 N solution). The resulting white solid (2.6 g) was filtered off and recrystallized from acetonitrile (40 ml) to give colorless crystals (2.1 g). In spite of giving a satisfactory elemental analysis, this material did not have a sharp melting point. It was, therefore, recrystallized from acetonitrile-isopropanol (4:1) to give sharply melting product, melting point 222.5-224°C.

Analysis Calc'd. for $C_{28}H_{29}NO_3 \cdot HCl$: C, 70.98; H, 6.87; N, 3.18

Found: C, 71.04; H, 6.94; N, 3.16

### Example 6

(trans)-3-[(2-Benzothiazolylmethyl)amino]-1,2,3,4-tetrahydro-5,8-dimethoxy-2-naphthalenol, hydrochloride salt (1:1)

A solution of 2-(aminomethyl)benzothiazole (1.7 g; 10 mmol), 6,7-epoxy-5,6,7,8-tetrahydro-1,4-dimethyoxynaphthalene (2.06 g; 10 mmol) and 3-pentanol (2.2 ml; ~20 mmol) in xylene (10 ml) was heated under reflux (oil bath 145°C) for 40

hours. The reaction had turned dark red by this time. It was allowed to cool to room temperature and the solvent was evaporated. The resulting dark thick oil was diluted with isopropanol-ether (25 ml 1:1 by volume) and treated with 5.5 N ethanolic hydrogen chloride (5.0 ml). A yellow precipitate was formed. The reaction was allowed to stand at room temperature for one hour and the precipitate was filtered off. The solid (2.1 g) obtained was recrystallized four times from acetonitrile-methanol (1 g of solid was heated with 15 ml of acetonitrile, and methanol was added dropwise until a clear solution was obtained which was allowed to cool) to give a tan solid (620 mg). Another recrystallization from isopropanol provided analytically pure product (435 mg) which decomposed at 208-212°C. This compound turned out to be a partial hydrate containing 0.66 mmol of water.

Analysis Calc'd. for $C_{20}H_{22}N_2S \cdot HCl \cdot 0.66H_2O$: C, 57.35; H, 5.85; N, 6.67; S, 7.65

Found: C, 57.35; H, 5.52; N, 6.27; S, 7.68

## Example 7

(trans)-3-[[(1H-Benzimidazol-2-yl)methyl]amino]-1,2,3,4-tetrahydro-5,8-dimethoxy-2-naphthalenol, hydrochloride salt (1:1)

A solution of 6,7-epoxy-5,6,7,8-tetrahydro-1,4-dimethoxynaphthalene (2.06 g; 10 mmol), 2-(aminomethyl)benzimidazole (2.3 g; 15 mmol, generated from its dihydrochloride) and 3-pentanol (2 ml) in xylene (4 ml) was heated under reflux (oil bath 145°C for 10 hours (TLC showed no starting material). The light brown reaction mixture was cooled to room temperature whereby it solidified. The solid was triturated with

acetonitrile and filtered off to give yellow crystalline product (2.1 g). The desired product was contaminated with a yellow impurity which had slightly higher $R_f$. Purification by recrystallization turned out to be difficult. Therefore, this material was suspended in ethanol-methanol (40 ml, 1:1) and treated with 5.5 N ethanolic hydrogen chloride (5.0 ml). The suspension became a homogeneous solution momentarily and then a colorless solid precipitated out. It was allowed to stand at room temperature for one hour and the solid (1.8 g) was filtered off and washed with ethanol and ether. Owing to its poor solubility in a variety of solvents, this material turned out to be impossible to purify by recrystallization. The yellow impurity, which was originally present in the product, was in fact removed during salt formation. In order to facilitate recrystallization (and also increase solubility of the product) the free amine had to be regenerated.

The salt of the product (1.8 g) was suspended in chloroform:methanol:water (100 ml:15 ml:50 ml) and 6N sodium hydroxide was added dropwise while stirring the two-phase reaction mixture vigorously. When the pH approached 12-13, most of the solid had disappeared. The organic layer was separated and the aqueous phase was saturated with sodium chloride and re-extracted with 5% methanolic chloroform (2x75 ml). The combined extracts were washed with brine and dried over magnesium sulfate. Evaporation provided a colorless soild (1.01 g) which was heated with acetonitrile (40 ml) and methanol was added dropwise until a clear solution was obtained. This solution was allowed to cool down to room temperature whereby a colorless

product crystallized out. It was filtered off and thoroughly washed with acetonitrile and chloroform to give the desired product as hard solid (502 mg), melting point 185.5-188.5°C.

Analysis Calc'd. for $C_{20}H_{23}N_3O_3$: C, 67.97; H, 6.56; N, 11.89

Found: C, 67.83; H, 6.74; N, 11.84

Example 8

(trans)-3-[(2-Benzothiazolylmethyl)amino]-1,2,3,4-tetrahydro-2-naphthalenol

A solution of 2-(aminomethyl)benzothiazole (325 mg; 1.83 mmol) in dichloromethane (2 ml) was treated with triethyl aluminum (0.96 ml of 1.9 M solution; 1.83 mmol) dropwise under an argon atmosphere. Effervescence was noted and the reaction turned deep purple. After stirring at room temperature for 30 minutes, a solution of 1,2-epoxy-1,2,3,4-tetrahydronaphthalene (262 mg; 1.8 mmol) in dichloromethane (1 ml) was added. The reaction was allowed to stir at room temperature over the weekend. It was diluted with 10 ml of dichloromethane and then treated with 6 N sodium hydroxide (3 ml). A slightly exothermic reaction took place. The resulting two-phase reaction mixture was stirred vigorously at room temperature for one hour. The reaction at this stage had turned deep yellow. The organic layer was separated and the aqueous phase was re-extracted with dichloromethane twice. The combined extracts were washed with brine, dried over anhydrous magnesium sulfate and evaporated to give a yellow solid. It was diluted with acetonitrile and filtered off to give light yellow crystalline material (201 mg). Recrystallization

from acetonitrile-chloroform (chloroform was added dropwise to the suspension of compound in hot acetonitrile, and the resulting solution was allowed to cool gradually) provided the analytically pure product (165 mg) as shiny crystals with yellow tinge, melting point 175.5-177.0°C.

Analysis Calc'd. for $C_{18}H_{18}N_2OS$: C, 69.95; H, 5.85; N, 9.02; S, 10.33

Found: C, 69.48; H, 5.87; N, 8.97; S, 10.08

## Example 9

(trans)-3-[[(Benzoxazol-2-yl)methyl]amino]-1,2,3,4-tetrahydro-5,8-dimethoxy-2-naphthalenol

Following the procedure of Example 8, but utilizing 2-(aminomethyl)benzoxazole (1.12 g; 7.54 mmol) in 5 ml of dichloromethane, triethyl aluminum (3.95 ml of a 1.9 M solution; 7.5 mmol), and 6,7-epoxy-5,6,7,8-tetrahydro-1,4-dimethoxynaphthalene (1.0 g; 4.9 mmol) in 4 ml of dichloromethane, yielded an oily residue. The residue was purified by flash chromatography on silica gel (5-6% methanol in chloroform) to give a colorless foam (1.03 g). This was dissolved in chloroform (5 ml) and then diluted with anhydrous ether (13 ml). The resulting solution was allowed to stand at room temperature overnight to give granular off-white crystals (750 mg). Another recrystallization from chloroform-ether gave analytically pure product, melting point 125-126°C.

Analysis Calc'd. for $C_{20}H_{22}N_2O_4$: C, 67.78; H, 6.26; N, 7.90

Found: C, 67.60; H, 6.39; N, 7.74

Example 10

(trans)-3-[[2-(2-Benzothiazolyl)ethyl]amino]-1,2,3,4-tetrahydro-5,8-dimethoxy-2-naphthalenol, hydrochloride salt (1:1)

Following the procedure of Example 8, but utilizing 2-(2-aminoethyl)benzothiazole (1.26 g; 7.08 mmol) in 6 ml of dichloromethane, triethyl aluminum (3.6 ml of 1.9 M sol; 7.0 mmol) and 6,7-epoxy-5,6,7,8-tetrahydro-1,4-dimethoxynaphthalene (1.24 g; 6 mmol) in 5 ml of dichloromethane, yielded 1.08 g, of a light yellow solid. This material turned out to be difficult to recrystallize. Therefore, it was dissolved in chloroform (10 ml) and treated with 5.5 N ethanolic hydrogen chloride (2.0 ml). The solution was allowed to stand at room temperature for 30 minutes, and the solvent was then evaporated to give a colorless solid. The solid was triturated with acetonitrile and filtered off (1.21 g). Recrystallization from isopropanol-methanol provided the analytically pure product, melting point 198.5-199.5°C.

Analysis Calc'd. for $C_{21}H_{24}N_2O_3S \cdot HCl$: C, 59.92; H, 5.99; N, 6.65; S, 7.62

Found: C, 59.91; H, 6.01; N, 6.60; S, 7.46

Example 11

(trans)-3-[(3,3-Dicyclohexylpropyl)amino]-1,2,3,4-tetrahydro-5,8-dimethoxy-2-naphthalenol, hydrochloride salt (1:1)

A solution of 3,3-dicyclohexylpropylamine (1.97 g; 8.84 mmol) in dry dichloromethane (10 ml) was treated with triethyl aluminum (4.6 ml of 1.9 M solution) under argon. After stirring at room temperature for 30 minutes, 6,7-epoxy-5,6,7,8-tetra-

hydro-1,4-dimethoxynaphthalene (1.82 g; 8.83 mmol) was added in dichloromethane (10 ml). The reaction was allowed to stir at room temperature overnight. It was diluted with 30 ml of dichloromethane, cooled to 0°C and treated dropwise with 6N sodium hydroxide (15 ml). The resulting two phase reaction mixture was vigorously stirred for 1 hour. The organic layer was separated and the aqueous layer was extracted twice with dichloromethane. The combined extracts were washed with brine, dried over anhydrous magnesium sulfate and evaporated to give a colorless foam. This material was dissolved in chloroform (25 ml) and treated with ethanolic hydrogen chloride (2.5 ml of 5N solution). After about 30 minutes, the solvent was evaporated to give a colorless solid. This was triturated with acetonitrile and filtered off to give colorless crystals (3.2 g). Recrystallization from isopropanol-methanol provided the analytically pure product, melting point 274-277°C (dec).

Analysis Calc'd. for $C_{27}H_{43}NO_3 \cdot HCl$: C, 69.58; H, 9.52; N, 3.01

Found: C, 69.86; H, 9.60; N, 3.04

## Example 12

**(trans)-3-[(3,3-Diphenylpropyl)amino]-1,2,3,4-tetrahydro-6,7-dimethoxy-2-naphthalenol, hydrochloride salt (1:1)**

A solution of 6,7-epoxy-5,6,7,8-tetrahydro-2,3-dimethoxynaphthalene (1.78 g; 7.8 mmol), 3,3-diphenylpropylamine (1.88 g; 8.6 mmol) and 3-pentanol (1.5 ml) in xylene (2.0 ml) was heated under reflux for 14 hours. The dark brown reaction was allowed to cool to room temperature whereby an off-white solid had separated. It was

diluted with anhydrous ether and the crystals were filtered off and washed with more ether. The product obtained (1.65 g) was taken up in chloroform (10 ml) and treated with ethanolic hydrogen chloride (3.5 ml of 5.5N solution). The solution was allowed to stand at room temperature for 1 hour and the solvent was then evaporated to give a colorless foam. Crystallization from isopropanol provided colorless crystalline product (1.75 g). An analytically pure sample was obtained by recrystallization from isopropanol, melting point 224.5-226°C. This compound turned out to be a partial hydrate containing 0.56 mole of water. Recrystallization from absolute ethanol or acetonitrile-methanol did not remove water from the crystals.

Analysis Calc'd. for $C_{27}H_{31}NO_3 \cdot HCl \cdot 0.56H_2O$: C, 69.87; H, 7.19; N, 3.02

Found: C, 69.87; H, 6.98; N, 3.06

## Example 13

(trans)-1,2,3,4-Tetrahydro-3-[[2-(1H-indol-3-yl)-ethyl]amino]-6,7-dimethoxy-2-naphthalenol

A solution of tryptamine (1.22 g, 7.61 mmol), recrystallized from chloroform/ethyl acetate, 6,7-epoxy-5,6,7,8-tetrahydro-2,3-dimethoxynaphthalene (1.57 g, 7.61 mmol) and 3-pentanol (2.5 ml) in sylene (4 ml) was refluxed under argon overnight. An off-white precipitate formed during this time. The reaction mixture was allowed to cool to ~85-90°C and acetonitrile (~6 ml) was added. The mixture was then left to cool to room temperature. The precipitate was filtered off and washed with acetonitrile and ether to give 2.1 g of an off-white solid. Recrystallization from

acetonitrile gave analytically pure off-white needles (1.85 g), melting point 180-182°C.
Analysis Calc'd. for $C_{22}H_{26}N_2O_3$: C, 72.11; H, 7.15; N, 7.64
Found: C, 72.10; H, 7.19; N, 7.82

Example 14

(trans)-1,2,3,4-Tetrahydro-5,8-dimethoxy-3-[[2-(6-methoxy-1H-indol-3-yl)ethyl]amino]-2-naphthalenol

A solution of 6,7-epoxy-5,6,7,8-tetrahydro-1,4-dimethoxynaphthalene (1.06 g; 5.15 mmol), 6-methoxytryptamine (980 mg; 5.15 mmol), and 3-pentanol (2.5 ml) in xylene (4 ml) was refluxed under argon for 6 hours. The reaction was allowed to cool slightly and was diluted with acetonitrile (15 ml). Only a small amount of material crystallized, so the mixture was rotary evaporated to a brown foam. Chromatography (silica gel, 2-4% methanol in dichloromethane) afforded 1.2 g of an off-white foam. Recrystallization from acetonitrile gave analytically pure off-white, granular crystals (608 mg), melting point 125-127°C.
Analysis Calc'd. for $C_{23}H_{28}N_2O_4$: C, 69.67; H, 7.12; N, 7.07
Found: C, 69.49; H, 7.11; N, 7.09

Example 15

(trans)-3-[[3-(2-Benzothiazolyl)propyl)amino]-1,2,3,4-tetrahydro-5,8-dimethoxy-2-naphthalenol, hydrochloride salt (1:1)

The reaction mixture containing 6,7-epoxy-5,6,7,8-tetrahydro-1,4-dimethoxynaphthalene (1.5 g; 7.3 mmol), benzothiazole-2-propanamine

(1.8 g, 9.4 mmol, crude) and 3-pentanol (1.5 ml) in xylene (3.0 ml) was heated under reflux for 16 hours. The solvent was evaporated to give a brown semi-solid. Purification by flash chromatography (2-3% methanol in dichloromethane) gave a light yellow foam (1.3 g). This was dissolved in chloroform (15 ml) and treated with 5.5N ethanolic hydrogen chloride (2 ml). After stirring at room temperature for 1 hour, the sovlent was evaporated to give a light yellow solid, which was recrystallized from isopropanol to give off white crystals (1.3 g). Another recrystallization from isopropanol-methanol provided the analytically pure product (melting point 250-252°C, dec.) as colorless fluffy material.

Analysis Calc'd. for $C_{22}H_{26}N_2O_3S \cdot HCl$: C, 60.75; H, 6.26; N, 6.44; S, 7.37

Found: C, 60.44; H, 6.19; N, 6.48; S, 7.40

## Example 16

(trans)-1,2,3,4-Tetrahydro-5,8-dimethoxy-3-[[2-(1H-indol-3-yl)ethyl]methylamino]-2-naphthalenol

A reaction mixture containing 6,7-epoxy-5,6,7,8-tetrahydro-1,4-dimethoxynaphthalene (1.2 g; 5.8 mmol), N-methyltryptamine (1.0 g; 5.8 mmol) and 3-pentanol (0.25 ml) was heated at 150°C (oil bath temp.) for 3 hours under argon. The light brown reaction was cooled to room temperature and was purified by flash chromatography (3-4% methanol in dichloromethane). The colorless foamy residue was crystallized from isopropyl ether-chloroform to give granular crystals (1.51 g). Recrystallization from isopropyl ether-chloroform provided the analytically pure product, melting point 155.5-156.5°C.

Analysis Calc'd. for $C_{23}H_{28}N_2O_3$: C, 72.60; H, 7.42; N, 7.36
Found: C, 72.55; H, 7.43; N, 7.26

Example 17

(trans)-1,2,3,4-Tetrahydro-5,8-dimethoxy-3-[[2-(5-methyl-1H-indol-3-yl)ethyl]amino]-2-naphthalenol

A solution of 5-methyltryptamine (1.47 g, 8.4 mmol), 6,7-epoxy-5,6,7,8-tetrahydro-1,4-dimethoxynaphthalene (1.73 g, 8.4 mmol), and 3-pentanol (3 ml) in xylene (5 ml) under argon was refluxed overnight. The reaction mixture was allowed to cool slightly and was diluted with acetonitrile (~5-10 ml). After about 10 minutes, an off-white precipitate formed. The mixture was allowed to stand for ca. 2 hours and then was filtered, yielding off-white crystals (2.0 g). This was recrystallized from acetonitrile to give analytically pure white crystals (1.37 g), melting point 141-143°C.
Analysis Calc'd. for $C_{23}H_{28}N_2O_3$: C, 72.60; H, 7.42; N, 7.36
Found: C, 72.72; H, 7.59; N, 7.41

Example 18

(trans)-3-[(4,4-Diphenylbutyl)amino]-1,2,3,4-tetrahydro-5,8-dimethoxy-2-naphthalenol, hydrochloride salt (1:1)

To a solution of 4,4-diphenylbutylamine (1.1 g) in dichloromethane (10 ml) under argon was added dropwise with stirring triethyl aluminum (1.90M solution in benzene, 2.1 ml, 4 mmol); effervescence was observed. The solution was allowed to stir for about one hour. 6,7-Epoxy-5,6,7,8-tetrahydro-1,4-dimethoxynaphthalene

(825 mg, 4.0 mmol) was added, and the resulting solution was allowed to stir overnight. In the morning, the reaction was cooled to 0°C and treated dropwise with 10 ml of 1N sodium hydroxide with vigorous stirring. After stirring for about one-half hour, the layers were separated and the aqueous layer was re-extracted with dichloromethane. The combined organic phases were washed with brine, dried (magnesium sulfate) and rotary evaporated to an oil (2.1 g). Flash chromatgraphy (3% methanol in dichloromethane with trace triethylamine) followed by treatment with excess ethanolic hydrogen chloride afforded white crystals (704 mg), melting point 259-261°C (dec). Recrystallization from ethanol/methanol gave the analytically pure white crystals (513 mg), melting point 261-264°C (dec).

Analysis Calc'd. for $C_{28}H_{33}NO_3 \cdot HCl$: C, 71.85; H, 7.11; N, 2.99

Found: C, 71.84; H, 7.37; N, 3.01

## Example 19

(trans)-3-[(4,4-Diphenylbutyl)amino]-1,2,3,4-tetrahydro-6,7-dimethoxy-2-naphthalenol, hydrochloride salt (1:1)

A solution of 6,7-epoxy-5,6,7,8-tetrahydro-2,3,-dimethoxynaphthalene (516 mg, 2.5 mmol), crude 4,4-diphenylbutylamine (1.1 g, <4.9 mmol) and 3-pentanol (0.5 ml) under argon was refluxed in an oil bath at 145-158°C. After 3 hours, TLC showed unreacted epoxide, so more of the crude amine (0.5 g) and 3-pentanol (0.5 ml) were added. After 4 more hours, epoxide still remained, so xylene (2 ml) was added, as well as more of the amine (0.3 g) and 3-pentanol (0.3 ml). The

reaction was allowed to reflux overnight. It was then diluted with toluene and rotary evaporated (three times) to yield an oil. Flash chromatography (dichloromethane → 5%, methanol in dichloromethane followed by treatment with excess ethanolic hydrogen chloride gave analytically pure white crystals (560 mg), melting point 165.5-167°C. Analysis Calc'd. for $C_{28}H_{33}NO_3 \cdot HCl$: C, 71.85; H, 7.11; N, 2.99

Found: C, 71.89; H, 7.44; N, 2.99

Example 20

(*trans*)-3-[(2,2-Diphenylethyl)amino]-1,2,3,4-tetrahydro-6,7-dimethoxy-2-naphthalenol, hydrochloride salt (1:1)

A mixture of 6,7-epoxy-5,6,7,8-tetrahydro-2,3-dimethoxynaphthalene (500 mg, 2.42 mmol), 2,2-diphenylethylamine (576 mg, 2.67 mmol) and 3-pentanol (0.5 ml) under argon was heated in an oil bath at 140-150°C. The reaction became homogeneous after less than a minute of heating. After 3.5 hours, the oil bath was removed and the reaction was allowed to cool slightly. Acetonitrile (5 ml) was added, and the solution was allowed to cool to room temperature. Crystals did not form. The reaction mixture was rotary evaporated to a brown oil, diluted with toluene and evaporated again. The resulting oil was chromatographed on silica (dichloromethane → 1%, methanol in dichloromethane with trace triethylamine) to give a foam. This was taken up in 20% isopropanol in acetonitrile (25 ml) treated with 5N ethanolic hydrogen chloride (5 ml), and allowed to stand overnight. Only a small amount of material crystallized, so the solvent was evaporated, and the resulting foam was triturated with

hexane/isopropyl ether/acetonitrile (approximately 1/1/1) to give white crystals (620 mg). Microanalysis showed the compound to be impure. Recrystallization from acetontrile gave analytically pure white crystals (312 mg), melting point 225-227°C.

Analysis Calc'd. for $C_{26}H_{29}NO_3 \cdot HCl$: C, 70.98; H, 6.87; N, 3.18

Found: C, 71.14; H, 6.95; N, 3.22

Example 21

(trans)-3-[(3,3-Diphenylpropyl)methylamino]-1,2,3,4-tetrahydro-6,7-dimethoxy-2-naphthalenol

The reaction mixture containing 6,7-epoxy-5,6,7,8-dimethoxynaphthalene (750 mg, 3.64 mmol), (3,3-diphenylpropyl)(methyl)amine (875 mg, 3.89 mmol) and 3-pentanol (0.5 ml) was heated under argon at 160-165°C (oil bath) for 9 hours; the reaction turned brown. It was cooled slightly and diluted with isopropanol (15 ml) and then allowed to cool to room temperature gradually. An off-white precipitate was formed which was filtered off and washed with more isopropanol and anhydrous ether. This material (1.3 g) was recrystallized twice from isopropanol to provide the title compound in analytically pure form (1.03 g), melting point 154-156°C.

Analysis Calc'd. for $C_{28}H_{33}NO_3$: C, 77.93; H, 7.71; N, 3.25

Found: C, 77.85; H, 7.63; N, 3.10

Example 22

(trans)-1,2,3,4-Tetrahydro-6,7-dimethoxy-3-[[3-(4-methoxyphenyl)-3-phenylpropyl]amino]-2-naphthalenol, hydrochloride salt (1:1)

A reaction mixture containing 6,7-epoxide-5,6,7,8-tetrahydro-2,3-dimethoxynaphthalene (690 mg, 3.35 mmol), [3-(4-methoxyphenyl)-3-phenylpropyl]amine (890 mg, 3.69 mmol) and 3-pentanol (0.5 ml) was heated at 160°C (oil bath temp.) for 4 hours. The reaction was allowed to cool slightly and diluted with isopropanol (15 ml). It was then allowed to cool down to room temperature whereby an off-white solid precipitated out. The light brown suspension was treated with ethanolic hydrogen chloride (3 ml of 5.5N solution). The solution became homogeneous momentarily and then a tan solid crystallized out. The solid was filtered off and washed with isopropanol and ether to give the desired product (1.3 g). This material was recrystallized twice from isopropanol-methanol to provide the analytically pure product (870 mg), melting point 194-202°C.

Analysis Calc'd. for $C_{28}H_{33}NO_4 \cdot HCl$: C, 68.33; H, 7.15; N, 2.85

Found: C, 68.33; H, 7.09; N, 2.85

## Example 23

(trans)-3-[(3,3-Diphenylpropyl)amino]-1,2,3,4-tetrahydro-6,7-dimethoxy-2-naphthalenol, monoacetate ester, hydrochloride salt (1:1)

A suspension of (trans)-3-[(3,3-diphenylpropyl)amino]-1,2,3,4-tetrahydro-6,7-dimethoxy-2-naphthalenol, hydrochloride salt (1:1) (700 mg, 1.54 mmol, see Example 12) in chloroform (15 ml) was treated with excess acetyl chloride (0.3 ml, 4 mmol) and the resulting heterogeneous reaction mixture was allowed to stir at room temperature under argon. After ~6 hours, the reaction became homogeneous. The stirring was continued overnight

and the solvent was then evaporated. The colorless oily residue was crystallized from isopropanol to give the title compound (698 mg), melting point 209-211.5°C.

Analysis Calc'd. for $C_{29}H_{33}NO_4 \cdot HCl$: C, 70.22; H, 6.91; N, 2.82

Found: C, 70.09; H, 6.91; N, 2.79

Example 24

(trans)-1,2,3,4-Tetrahydro-6,7-dimethoxy-3-[(4-methyl-3-phenylpentyl)amino]-2-naphthalenol, hydrochloride salt (1:1)

A solution of (4-methyl-3-phenylpentyl)amine (500 mg, 2.8 mmol), 6,7-epoxy-5,6,7,8-tetrahydro-2,3-dimethoxynaphthalene (618 mg, 3.0 mmol) and 3-pentanol (0.2 ml) under argon was refluxed in an oil bath at 160°C for 3 hours. The reaction mixture was allowed to cool to room temperature and was co-evaporated with tolune a few times. The oil obtained was chromatographed (silica, 4% methanol in dichloromethane) to yield, again, an oil. This was taken up in 10% acetonitrile in ether and treated with 5N ethanolic hydrogen chloride (2 ml). Analytically pure white crystals were obtained (794 mg). This material was combined with 200 mg from another run and recrystallized from isopropanol/methanol to give analytically pure white crystals (621 mg), melting point 243-245°C (dec).

Analysis Calc'd. for $C_{24}H_{33}NO_3 \cdot HCl$: C, 68.64; H, 8.16; N, 3.33

Found: C, 68.32; H, 8.14; N, 3.34

Example 25

(trans)-3-[2-(9H-Fluoren-9-yl)ethyl]amino]-1,2,3,4-tetrahydro-6,7-dimethoxy-2-naphthalenol, hydrochloride salt (1:1)

A solution of [2-(9H-fluoren-9-yl)ethyl]-amine (630 mg, 30 mmol) and 6,7-epoxy-5,6,7,8-tetrahydro-2,3-dimethoxynaphthalene (516 mg, 2.5 mmol) in 3-pentanol (0.2 ml) under argon was heated in an oil bath at 160°C for 3 hours. The reaction mixture was allowed to cool to room temperature and was co-evaporated several times with toluene. The oily residue was chromatographed (silica, 4% methanol in acetonitrile) to give, again, an oil. This was taken up in chloroform/isopropanol/ether and treated with excess ethanolic hydrogen chloride (5N). Off-white crystals were obtained (785 mg). Recrystallization from methanol gave analytically pure colorless crystals (582 mg), melting point: decomposes >275°C.

Analysis Calc'd. for $C_{27}H_{29}NO_3 \cdot HCl$: C, 71.75; H, 6.47; N, 3.10

Found: C, 71.90; H, 6.70; N, 3.23

Example 26

(trans)-3-[(5,5-Diphenylpentyl)amino]-1,2,3,4-tetrahydro-5,8-dimethoxy-2-naphthalenol, hydrochloride salt (1:1)

A mixture of 6,7-epoxy-5,6,7,8-tetrahydro-1,4-dimethoxynaphthalene (0.95 g, 4.62 mmol), 5,5-diphenylpentylamine (1.15 g, 4.76 mmol) and 0.2 ml of 3-pentanol was heated under argon (oil bath temperature 160°C) for 3.5 hours. After

cooling for ca. five minutes, 7 ml of isopropanol were added to induce crystallization. Filtration of the reaction mixture yielded a mixture of a solid and a tar (1.95 g). Flash chromatography (LPS-1, 200 g) of the crude amine yielded the title compound as a light brown oil (0.57 g). Complete elution of the title compound required dichloromethane/methanol solutions with increasing amounts of methanol until 100% methanol was used. Also eluted from the column was 0.53 g of material with an $R_f$ similar to that of the starting epoxide (silica, 90/10 dichloromethane/methanol). TLC of the title compound (silica, 90/10 dichloromethane/methanol) indicated the presence of several impurities. The crude product was dissolved in isopropanol and acidified with 6M hydrogen chloride; removal of the isopropanol/water *in vacuo* yielded a brown solid which was recrystallized from 95% ethanol to yield 0.24 g of an off-white crystalline solid. Attempts at recrystallization from other solvent systems may have caused some decomposition, decreasing the yield.

Analysis Calc'd. for $C_{29}H_{35}NO_3 \cdot HCl \cdot 0.41H_2O$: C, 71.17; H, 7.58; N, 2.86

Found: C, 71.17; H, 7.55; N, 2.89

## Example 27

**(*trans*)-3-[(3,3-Diphenylpropyl)amino]-1,2,3,4-tetrahydro-6,7-dimethoxy-2-naphthalenol, lactate salt (1:1)**

(*trans*)-3-[(3,3-diphenylpropyl)amino]-1,2,3,4-tetrahydro-6,7-dimethoxy-2-naphthalenol (4.1 g, 9183 mmol, regenerated from the hydrochloride salt of Example 12) in chloroform (15 ml)

was treated with the solution of 80% lactate acid (1.61 g, 14.4 mmol, dl) in the same solvent (5 ml). After about 10 minutes, anhydrous ether was added slowly until the solution became slightly cloudy. On swirling the flask, colorless crystals of the lactic acid salt were formed. More anhydrous ether was added until most of the product crystallized out. The solid was filtered off and washed with ether to give colorless crystallizatine salt (4.9 g). Microanalysis showed this material to be deficient in carbon. The product was dried under vacuum at 65°C overnight but the analysis showed no improvement. The NMR (270 MHz) revealed the presence of some ether in the sample. The product was recrystallized from acetonitrile (50-60 ml is needed to dissolve 1 g of the salt) to give analytically pure product (4.46 g), melting point 175-176.5°C.

Analysis Calc'd. for $C_{27}H_{31}NO_3 \cdot C_3H_6O_2$: C, 70.98; H, 7.35; N, 2.76

Found: C, 71.10; H, 7.33; N, 2.82

Example 28

(trans)-N-(3,3-Diphenylpropyl)-1,2,3,4-tetrahydro-3,6,7-trimethoxy-2-naphthalenamine, oxalate salt (1:1)

A suspension of potassium hyride (74 mg of 24% suspension in oil, 1.83 mmol) in anhydrous tetrahydrofuran (1 ml) was cooled to 0°C and treated with the solution of (trans)-3-[(3,3-diphenylpropyl)amino]-1,2,3,4-tetrahydro-6,7-dimethoxy-2-naphthalenol (700 mg, 1.7 mmol, regenerated from the hydrochloride salt of Example 12) in tetrahydrofuran-dimethylformamide (12 ml, 5:1). After the addition was over, the reaction

was allowed to warm to room temperature and stirring was continued for 30 minutes. It was then cooled to 0°C and treated dropwise with methyl iodide (260 mg, 1.83 mmol). The reaction was allowed to stir at room temperature for 3 hours and then quenched with methanol (2 ml) followed by water (5 ml). The reaction was extracted with chloroform, and the extracts were washed with water (3 x 15 ml), brine and dried over anhydrous potassium carbonate. Evaporation provided a pale yellow oil which was purified by flash chromatography on silica gel (2% methanol in chloroform) to provide the desired product (449 mg). Some starting material (152 mg) was also recovered from the column. The desired product did not crystallize from a variety of solvent systems. It was, therefore, converted to its hydrochloride by treatment with ethanolic hydrogen chloride (1 ml of 5.5 N solution). The hydrochloride also turned out to be exceedingly difficult to recrystallize. The free amine was regenerated (chloroform solution was washed with 2N sodium hydroxide) and purified again by flash chromatography (2% methanol in chloroform) to give colorless foam (360 mg). It was dissolved in isopropanol (5 ml) and treated with oxalic acid (75 mg). After about 30 minutes, a white solid had separated. The solvent was stripped off, and the product was recrystallized from acetone to give colorless crystals (301 mg), melting point 186-188°C.

Analysis Calc'd. for $C_{28}H_{33}NO_3 \cdot C_2H_2O_4$: C, 68.22; H, 6.82; N, 2.65

Found: C, 68.22; H, 6.82; N, 2.65

Example 29

(cis)-3-[(3,3-Diphenylpropyl)amino]-1,2,3,4-tetrahydro-6,7-dimethoxy-2-naphthalenol, hydrochloride salt (1:1)

A) (trans)-3-(Benzoylamino)-1,2,3,4-tetrahydro-6,7-dimethoxy-2-naphthalenol

To a solution of (trans)-3-amino-1,2,3,4-tetrahydro-6,7-dimethoxy-2-naphthalenol (1.30 g, 5.0 mmol) in water (15 ml) at 0°C was added a solution of benzoyl chloride (0.70 g, 5.0 mmol) in toluene (5 ml). To this vigorously stirred two-phase mixture was added, over the course of ~2 hours, sufficient 1N aqueous sodium hydroxide to keep the pH at 7-9. (A white precipitate formed shortly after the first addition of sodium hydroxide solution). The mixture was diluted with chloroform and 1N sodium hydroxide and shaken in a separatory funnel. The precipitate would not dissolve, however, so the mixture was filtered and the filter cake washed with water. The white solid obtained was recrystallized from acetonitrile to give white crystals, 1.30 g, melting point 209-211°C. A second crop was obtained (white crystals, 176 mg, melting point partial at 191-193°C, cmplete at 209-211°C) that was identical by TLC. Combined yield: 1.48 g.

B) (cis)-3-Amino-1,2,3,4-tetrahydro-6,7-dimethoxy-2-naphthalenol, hydrochloride salt (1:1)

A solution of (trans)-3-(benzoylamino)-1,2,3,4-tetrahydro-6,7-dimethoxy-2-naphthalenol (1.15 g, 3.52 mmol) in thionyl chloride (0.7 ml) under argon was heated in an oil bath at 50°C for

2 hours. The brown reaction mixutre was diluted with toluene, and a colorless precipitate formed. The solvent was rotary evaporated, leaving a pale orange solid. A suspension of the solid in 2.5N aqueous hydrogen chloride was refluxed overnight, and the solid gradually dissolved. The solution was allowed to cool and two layers formed. The solvent was rotary evaporated, and the oily residue was triturated with acetonitrile to give an off-white solid (672 mg).

C) 3,3-Diphenylpropionaldehyde

To a suspension of pyridinium chlorochromate (1.30 g, 6.0 mmol) in dry dichloromethane (8 ml) under argon was added a solution of 3,3-diphenyl-1-propanol (0.851 g, 4.0 mmol) in dry dichloromethane (4 ml). The mixture darkened and became homogeneous. After a few minutes, a black gum began to precipitate. The reaction mixture was allowed to stir for ~2 hours, and then was diluted with ether (50 ml) and suction filtered through a sintered glass funnel containing a pad of Fluorisil under a pad of Celite. The filter pad was rinsed with ether (3 x 10 ml), and the filtrate was evaporated to a nearly colorless oil (727 mg).

D) (cis)-3-[(3,3-Diphenylpropyl)amino]-1,2,3,4-tetrahydro-6,7-dimethoxy-2-naphthalenol, hydrochloride salt (1:1)

To a solution of (cis)-3-amino-1,2,3,4-tetrahydro-6,7-dimethoxy-2-naphthalenol, hydrochloride salt (1:1) (622 mg, 2.4 mmol) in dry methanol (5 ml) under argon was added triethylamine (0.21 ml, 1.5 mmol) followed by a solution of 3,3-diphenylpropionaldehyde (626 mg, 3.0 mmol)

in dry methanol (5 ml). Sodium cyanoborohydride (280 mg, 4.5 mmol, Aldrich) was then added, and the solution was allowed to stir at room temperature for two days. The solution was then cooled in an ice water bath and treated dropwise with concentrated hydrochloric acid until the pH was 2. The solvent was rotary evaporated leaving a colorless solid. To the solid were added ice, chloroform, and 1N aqueous sodium hydroxide, and this mixture was shaken until the solid dissolved. The layers were separated, and the aqueous phase was extracted again with chlorform. The combined organic extracts were washed with brine, dried (anhydrous potassium carbonate), filtered, and rotary evaporated to an oil. The oil was chromatographed on silica (dichloromethane/methanol/acetic acid, 100/1/1) followed by 4% methanol in dichloromethane to give an oil. This was taken up in dichloromethane, washed with 1N sodium hydroxide, brine, dried (potassium carbonate), filtered and treated with excess methanolic hydrogen chloride. The solvent was evaporated, and the colorless solid residue was recrystallized from isopropanol/chloroform/methanol to give analytically pure white crystals (388 mg), melting point 257.5-260°C (dec). [Yield of this experiment can be increased by recrystallization of the intermediate of part B from acetonitrile prior to its use in the next step].

Analysis Calc'd. for $C_{27}H_{31}NO_2 \cdot HCl$: C, 71.43; H, 7.10; N, 3.09; Cl, 7.81

Found: C, 71.44; H, 7.20; N, 2.98; Cl, 7.76

CLAIMS

1. A compound having the formula

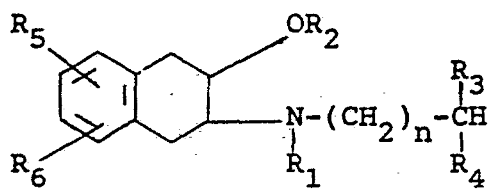

or a pharmaceutically acceptable salt thereof, wherein

$R_1$ is hydrogen or methyl;

$R_2$ is hydrogen, alkyl, alkanoyl, or arylcarbonyl;

$R_3$ and $R_4$ are each aryl; $R_3$ and $R_4$ are each cycloalkyl; $R_3$ is hydrogen or alkyl and $R_4$ is cycloalkyl or 9H-fluoren-9-yl; or $R_3$ is hydrogen or alkyl and $R_4$ is heteroaryl;

$R_5$ and $R_6$ are the same or different and each is hydrogen, hydroxy, alkoxy or alkanoyl; and

n is 1, 2, 3 or 4;

wherein the terms "alkyl" and "alkoxy" refer to groups having 1 to 7 carbon atoms;

the term "cycloalkyl" refers to groups having 3, 4, 5, 6 or 7 carbon atoms;

the term "alkanoyl" refers to groups having 2 to 7 carbon atoms;

the term "aryl" refers to phenyl and phenyl substituted with 1, 2 or 3 alkyl, alkoxy, halogen, alkylamino, dialkylamino, alkanoylamino or arylcarbonylamino groups; and

the term "heteroaryl" refers to 2-, 3- or 4-pyridinyl, 2- or 3-furanyl, 2- or 3-thienyl, 1-, 2- or 3-indolyl, 2-benzoxazolyl, 2-benzothiazolyl, 1- or 2-benzimidazolyl, 2- or 3-benzothienyl and 2- or 3-benzofuranyl, and to any of the above groups substituted with 1, 2 or 3 alkyl, alkoxy, halogen,

alkylamino, dialkylamino, alkanoylamino, or arylcarbonylamino groups.

2. A compound in accordance with Claim 1 having the formula

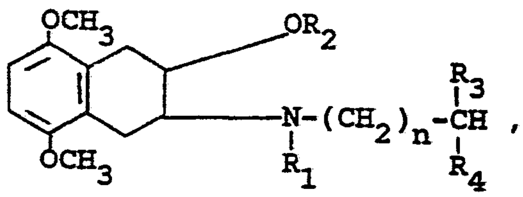

or a pharmaceutically acceptable salt thereof.

3. A compound in accordance with Claim 1 having the formula

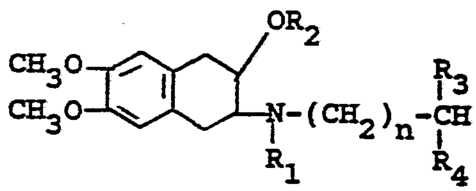

or a pharmaceutically acceptable salt thereof.

4. A compound in accordance with Claim 1, 2 or 3 wherein $R_2$ is hydrogen.

5. A compound in accordance with Claim 1, 2, 3 or 4 wherein $R_3$ and $R_4$ are each aryl.

6. A compound in accordance with Claim 1, 2, 3 or 4 wherein $R_3$ and $R_4$ are each phenyl.

7. A compound in accordance with Claim 1, 2, 3 or 4 wherein $R_3$ and $R_4$ are each cycloalkyl.

8. A compound in accordance with Claim 1, 2, 3 or 4 wherein $R_3$ and $R_4$ are each cyclohexyl.

9. A compound in accordance with Claim 1, 2, 3 or 4 wherein $R_3$ is hydrogen or alkyl, and $R_4$ is cycloalkyl or 9H-fluoren-9-yl.

10. A compound in accordance with Claim 1, 2, 3 or 4 wherein $R_3$ is hydrogen or alkyl, and $R_4$ is heteroaryl.

11. The compound in accordance with claim 1, (trans)-3-[(3,3-dicyclohexylpropyl)amino]-1,2,3,4-tetrahydro-5,8-dimethoxy-2-naphthalenol, or a pharmaceutically acceptable salt thereof.

12. The compound in accordance with claim 1, (trans)-3-[(3,3-diphenylpropyl)amino]-1,2,3,4-tetrahydro-6,7-dimethoxy-2-naphthalenol, or a pharmaceutically acceptable salt thereof.

13. A process for preparing a compound having the formula

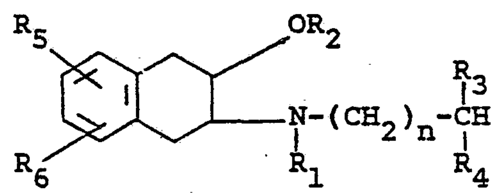

,

or a pharmaceutically acceptable salt thereof, wherein

$R_1$ is hydrogen or methyl;

$R_2$ is hydrogen, alkyl, alkanoyl, or arylcarbonyl;

$R_3$ and $R_4$ are each aryl; $R_3$ and $R_4$ are each cycloalkyl; $R_3$ is hydrogen or alkyl and $R_4$ is cycloalkyl or 9H-fluoren-9-yl; or $R_3$ is hydrogen or alkyl and $R_4$ is heteroaryl;

$R_5$ and $R_6$ are the same or different and each is hydrogen, hydroxy, alkoxy or alkanoyl; and

n is 1, 2, 3 or 4;

wherein the terms "alkyl" and "alkoxy" refer to groups having 1 to 7 carbon atoms;

the term "cycloalkyl" refers to groups having 3, 4, 5, 6 or 7 carbon atoms;

the term "alkanoyl" refers to groups having 2 to 7 carbon atoms;

the term "aryl" refers to phenyl and phenyl substituted with 1, 2 or 3 alkyl, alkoxy, halogen, alkylamino, dialkylamino, alkanoylamino or arylcarbonylamino groups; and

the term "heteroaryl" refers to 2-, 3- or 4-pyridinyl, 2- or 3-furanyl, 2- or 3-thienyl, 1-, 2- or 3-indolyl, 2-benzoxazolyl, 2-benzothiazolyl, 1- or 2-benzimidazolyl, 2- or 3-benzothienyl and 2- or 3-benzofuranyl, and to any of the above groups substituted with 1, 2 or 3 alkyl, alkoxy, halogen, alkylamino, dialkylamino, alkanoylamino, or arylcarbonylamino groups which comprises either

(a) reacting a 1,2,3,4-tetrahydro-6,7-epoxynaphthalene of the formula

II

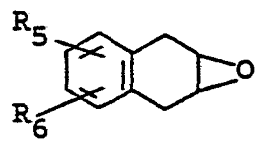

with an amine of the formula

III

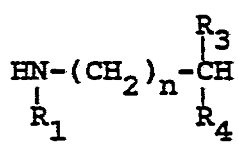

to yield a compound of the formula

IV

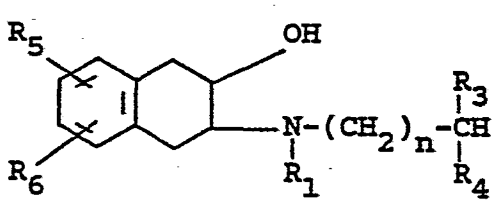

and,

optionally, converting to compounds wherein $R_2$ is alkyl, alkanoyl or arylcarbonyl by known methods; or (b) reacting the cis isomer of a compound of the formula

V 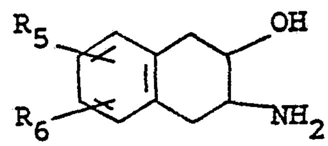

with a compound of the formula

VI 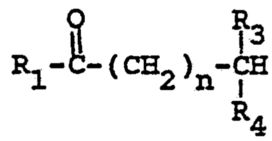 .

European Patent Office

# EUROPEAN SEARCH REPORT

0163458
Application number

EP 85303472.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | US - A - 4 267 373 (HAUCK et al.)<br>* Example 8 * | 1,13 | C 07 C 93/14<br>C 07 C 91/23<br>C 07 D 209/16<br>C 07 D 277/64<br>C 07 D 235/14<br>C 07 D 263/56<br>//A 61 K 31/135 |
| D,A | US - A - 3 930 022 (HAUCK et al.)<br>* Example 2 * | 1,13 | |
| A | GB - A - 1 442 851 (SQUIBB & SONS)<br>* Claims 1,3,7; examples 3,8 * | 1,13 | |
| A | GB - A - 1 516 677 (TAKEDA YAKUHIN)<br>* Claims 1,7; examples 15,48, 87 * | 1,10, 13 | |
| A | PATENT ABSTRACTS OF JAPAN, unexamined applications, Section C, vol. 2, Nr. 48, March 31, 1978<br>THE PATENT OFFICE JAPANESE GOVERNMENT<br>page 128 C 78<br>* Kokai-no. 53-7 658 (TAKEDA YAKUHIN) * | 1 | **TECHNICAL FIELDS SEARCHED (Int. Cl.4)**<br>C 07 C 93/00<br>C 07 C 91/00<br>C 07 D 209/00<br>C 07 D 277/00<br>C 07 D 235/00<br>C 07 D 263/00 |

The present search report has been drawn up for all claims

| Place of search | Date of completion of the search | Examiner |
|---|---|---|
| VIENNA | 05-08-1985 | KÖRBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82